(19) Europäisches Patentamt
European Patent Office
Office européen des brevets

(11) Publication number: 0 187 019 B1

(12) EUROPEAN PATENT SPECIFICATION

(45) Date of publication of patent specification: 14.11.90

(51) Int. Cl.[5]: C 07 C 215/52, C 07 C 59/01

(21) Application number: 85309216.1

(22) Date of filing: 18.12.85

# (54) Improvements in or relating to dobutamine salts.

(30) Priority: 20.12.84 US 684404

(43) Date of publication of application: 09.07.86 Bulletin 86/28

(45) Publication of the grant of the patent: 14.11.90 Bulletin 90/46

(84) Designated Contracting States: AT BE CH DE FR GB IT LI LU NL SE

(56) References cited:
US-A-3 987 200
US-A-4 175 136

(73) Proprietor: ELI LILLY AND COMPANY
Lilly Corporate Center
Indianapolis Indiana 46285 (US)

(72) Inventor: Massey, Eddie Herman
8337 HiVu Drive
Indianapolis, IN 46227 (US)

(74) Representative: Tapping, Kenneth George et al
Erl Wood Manor
Windlesham Surrey, GU20 6PH (GB)

The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

Dobutamine, N-[3-(4-hydroxyphenyl)-1-methylpropyl]-2-(3,4-dihydroxyphenyl)ethylamine is marketed as the hydrochloride salt. Its use as an ionotrope for treating cardiac insufficiency is disclosed in United States Patent No. 3,987,200. This U.S. patent discloses mineral salts of dobutamine, including the hydrochloride. Unfortunately, such salts of dobutamine are substantially insoluble in water. US—A—4175136 discloses a number of salts of N-phenethyl-N-propyl-3,4-dihydroxyphenethylamines.

In accordance with the invention, it has now been discovered that salts of dobutamine formed with an acid of formula I:

$$(OH)_{1-8}-(C_{2-11}\ alkyl)-(COOH)_{1-2} \qquad I$$

are water-soluble and that pharmaceutical formulations containing such salts have improved stability characteristics.

Examples of such acids include lactic acid, gluconic acid, lactobionic acid, glucoheptonic acid, glyceric acid, glycollic acid, tartaric acid, malic acid, mevalonic acid, dihydroxybutyric acid, dihydroxyisobutyric acid, dihydroxyvaleric acid, dihydroxy isovaleric acid, erythronic acid, threonic acid, bis(hydroxymethyl)malonic acid, 2,3-dihydroxy glutaric acid, dihydroxyadipic acid, bis(hydroxymethyl) acetic acid and the like. Salts of dobutamine formed with optical isomers of the above acids are included within the scope of this invention.

Dobutamine is a racemate of the following structure:

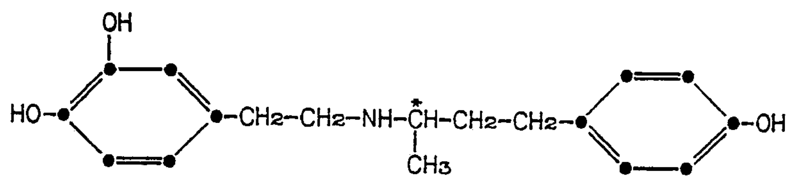

The α-carbon (marked with an asterisk) is asymmetric, yielding two stereoisomers, the (+) and the (−), which, in a 1:1 mixture, form the racemate. This invention includes soluble salts formed with acids of formula I of (+)-N-[3-(4-hydroxyphenyl)-1-methylpropyl]-2-(3,4-dihydroxyphenyl)ethylamine and of the corresponding (−) stereoisomer as well as of the racemate. It will be understood that the term dobutamine as used herein includes both the racemate and the stereoisomers.

The soluble dobutamine salts of this invention can be prepared by direct neutralization of the amine base or by a metathetic reaction (double decomposition) involving an acid addition salt of dobutamine. The neutralization procedure is preferred.

According to one aspect of the invention there is provided a process for preparing a soluble salt of dobutamine and an acid of formula:

$$(OH)_{1-8}-(C_{2-11}\ alkyl)-(COOH)_{1-2} \qquad I$$

which comprises:

(a) neutralizing dopamine as the free base with the acid of formula (I), or

(b) the metathetic reaction of an acid addition salt of dobutamine with a salt of the acid of formula (I).

In the neutralization procedure, dobutamine free base can be prepared by suspending dobutamine hydrochloride in oxygen-free water, adding an antioxidant (peroxide scavenger) followed by the addition of a dilute aqueous solution of a base (an inorganic base such as an alkali metal hydroxide, carbonate, bicarbonate etc. or a water-soluble organic base such as tris(hydroxymethyl)methylamine). Dobutamine base is water-insoluble and separates as a white crystalline mass from the alkaline medium. The crystals are separated, as by filtration, and dried. The dried crystals are then added to oxygen free water containing an antioxidant. The desired acid (for salt formation) is added in slight excess as an aqueous solution, and the desired salt is formed. The salt solution can be stored for filling into vials where the solution is lyophilized or the salt solution can first be lyophilized and the lyophilized salt stored.

All the above operations are carried out in an oxygen-free (insofar as practicable) atmosphere.

A typical example of such a reaction sequence follows.

Example 1

Preparation of Dobutamine Salts

Seventeen grams of dobutamine hydrochloride were suspended in 500 ml of deaerated water [deaerated by alternatively applying a vacuum (10—100 Torr.) followed by a period of sparging with $N_2$]. 150 mg of sodium sulfite were added as a peroxide scavenger. The suspension was stirred under a constant positive $N_2$ pressure. 50 ml of 1N aqueous sodium hydroxide diluted to 120 ml with deaerated water were added in dropwise fashion over a 2.5 hr. period. After the addition was complete, the

neutralization mixture was stirred for an additional 2 hours, during which time a white crystalline solid comprising dobutamine free base separated. The supernate was decanted, and the crystalline residue washed thoroughly with 600 ml of deaerated water. The supernate was again decanted. The solid precipitate was then filtered under $N_2$. The filter cake was washed twice with 100 ml portions of water and was then dried at 45°C under vacuum for 3 hours. 14.5 g of dobutamine free base were thus obtained.

A repeat preparation was carried out on a 3x scale except that sodium bisulfite (0.5 g) was used in place of sodium sulfite; yield of dried crystalline dobutamine free base = 43.0 g (96%).

8.3 Millimoles of dobutamine free base and 8.7 millimoles of each of the following acids: glycolic, (+)-tartaric, gluconic, lactobionic and glucoheptonic, were mixed under an $N_2$ atmosphere with an amount of 0.035 g aqueous sodium bisulfite dissolved in water for injection such that the final volume was 25 ml; i.e., 100 mg/ml of dobutamine base. If required, the salt-forming mixture was warmed to expedite solution of dobutamine base. The solid salt can be obtained from the solution, if desired, by evaporation of the solvent.

The solubilities of each of the dobutamine salts thus prepared was in excess of 100 mg/ml. By contrast, a saturated solution of dobutamine hydrochloride provides about 25 mg/ml of dobutamine.

In a repeat with (−)-tartaric acid, 58.6 g of dobutamine hydrochloride in deaerated water were neutralized with dilute aqueous sodium hydroxide in the presence of a peroxide scavenger under $N_2$; pH = 11.09. The precipitate of dobutamine base was washed with the aerated water and dried. The filter cake was dissolved in 2 l of deaerated water followed by 26.1 g of (−)-tartaric acid and 0.8 g of sodium bisulfite; pH = 3.65. The solution was filtered and then stored at room temperature, always under an $N_2$ atmosphere. Such stored concentrate solutions are stable for 12 months or more at 5°C.

A preferred method of preparing dobutamine free base involves the use of tris(hydroxymethyl)-methylamine in place of aqueous sodium hydroxide. A typical preparation using this procedure is carried out as follows: 500 ml of deoxygenated water for injection (WFI) are placed in a 2 l 3-neck round-bottom flask equipped with $N_2$ inlet, dropping funnel, stirring means and a probe leading to a pH meter. A nitrogen sweep is maintained throughout the operation. 50 mg of $NaHSO_3$ are added and the mixture stirred till solution occurs. 80.16 g of dobutamine hydrochloride are added via a y-tube adapter, and any salt remaining in the addition tube is washed in with an additional 500 ml of deoxygenated water (WFI). The resulting slurry is stirred for 15—20 minutes. 250 ml of 1M aqueous tris(hydroxymethyl)methylamine [30.3 g in 250 ml of deoxygenated water (WFI)] are added in dropwise fashion over a 1—2 hr. period at such a rate that the pH of the solution does not rise above pH = 8.5. After the addition is completed, the neutralization mixture is chilled to a temperature in the range 0—10°C. The chilled solution is then vacuum filtered under $N_2$. The filter cake comprising dobutamine free base is washed with 1200 ml of deoxygenated water (WFI) (in three 400 ml portions). Next, a solution is prepared in a 2 l beaker by dissolving 35.56 g of (+)-tartaric acid and 1.3 g of $NaHSO_3$ in 1 l of deoxygenated water (WFI). The filter cake is added with stirring to this solution under $N_2$. After the dobutamine free base has all dissolved, the solution is assayed for dobutamine by UV and the volume of solution now containing the (+)-tartrate salt is adjusted by adding deoxygenated water (WFI) such that the final dobutamine concentration is 50 mg./ml.

Specific rotation of the (+)-tartrate salt

$$[\alpha]_D^{25} = +8.8°.$$

Five milliliter aliquots are then transferred under $N_2$ to a series of vials. The vials under $N_2$ can be sealed as such or lyophilized and then sealed.

Alternatively, dobutamine free base (the filter cake) can be stored as such and converted to the tartrate salt (or other salt) at a later time in a different area.

In the above preparations, argon can be used in place of nitrogen. In addition, other antioxidants may be used in place of sodium sulfite or sodium bisulfite such as other alkali metal sulfites or bisulfites, thioglycerol, thioerithritol etc.

Although (+)-tartaric and (−)-tartaric acids were employed above, (±)-tartaric and mesotartaric acids are also fully operative and form dobutamine salts of high solubility. Furthermore, with any of the acids named, stereoisomers thereof can also be used rather than a racemate. Dobutamine content of all solutions is monitored with UV.

A typical injectable (i.v.) formulation using dobutamine tartrate has the following composition:

250 mg (base equivalent) dobutamine tartrate
4 mg sodium bisulfite
water q.s. to 5 ml.

Such solutions are placed in 5 ml glass ampoules and the ampoule sealed while maintaining an oxygen-free atmosphere. Glass vials with rubber stoppers can be used as well. The ampoules can be opened as needed to provide an i.v. injectable solution for treatment of cardiogenic shock. Alternatively, the solutions can be lyophilized and reconstituted with WFI prior to use.

Formulations containing dobutamine salts prepared as above have superior stability characteristics. Accordingly, in one aspect of the invention there is provided a pharmaceutical formulation containing an aqueous solution of a dobutamine salt of an acid of formula I (preferably the (+)-tartrate) and an antioxidant (preferably sodium bisulphite).

**Claims for the Contracting States: BE CH DE FR GB IT LI LU NL SE**

1. A salt of N-[3-(4-hydroxyphenyl)-1-methylpropyl]-2-(3,4-dihydroxyphenyl)ethylamine with an acid of the formula I:

$$(OH)_{1-8}(C_{2-11}\ alkyl)(COOH)_{1-2} \qquad I$$

or with lactobionic acid or glycolic acid.

2. Dobutamine tartrate.

3. Dobutamine (+)-tartrate.

4. Dobutamine lactobionate.

5. Dobutamine lactate.

6. Solid, crystalline, filterable dobutamine free base.

7. A pharmaceutical formulation comprising an aqueous solution of a dobutamine salt according to any one of claims 1 to 5, associated with an antioxidant.

8. A pharmaceutical formulation comprising an aqueous solution of dobutamine (+)-tartrate and sodium bisulphite.

9. A pharmaceutical formulation comprising an aqueous solution of dobutamine lactobionate.

10. A pharmaceutical formulation comprising an aqueous solution of dobutamine lactate.

11. A process for preparing a salt of dobutamine and an acid of formula:

$$(OH)_{1-8}(C_{2-11}\ alkyl)(COOH)_{1-2} \qquad I$$

or lactobionic acid or glycolic acid which comprises:
(a) neutralizing dobutamine as the free base with the acid, or
(b) the metathetic reaction of an acid addition salt of dobutamine with a salt of the acid.

**Claims for the Contracting State: AT**

1. A process for preparing a salt of dobutamine and an acid of formula:

$$(OH)_{1-8}(C_{2-11}\ alkyl)(COOH)_{1-2} \qquad I$$

or with lactobionic acid or glycolic acid which comprises:
(a) neutralizing dobutamine as the free base with the acid, or
(b) the metathetic reaction of an acid addition salt of dobutamine with a salt of the acid.

2. A process according to claim 1, wherein the acid of formula (I) is tartaric acid.

3. A process according to claim 2, wherein the acid of formula (I) is (+)-tartaric acid.

4. A process according to claim 1, wherein the acid is lactobionic acid.

5. A process according to claim 1, wherein the acid is lactic acid.

**Patentansprüche für die Vertragsstaaten: BE CH/LI DE FR GB IT LU NL SE**

1. Salz von N-[3-(4-Hydroxyphenyl)1-methylpropyl]-2-(3,4-dihydroxyphenyl)ethylamin mit einer Säure der Formel I

$$(OH)_{1-8}(C_{2-11}\ Alkyl)(COOH)_{1-2} \qquad I$$

oder mit Lactobionsäure oder Glycolsäure.

2. Dobutamintartrat.

3. Dobutamin-(+)-tartrat.

4. Dobutaminlactobionat.

5. Dobutaminlactat.

6. Festes, kristallines, filtrierbares Dobutamin in Form der freien Base.

7. Pharmazeutische Formulierung aus einer wäßrigen Lösung eines Dobutaminsalzes nach einem der Ansprüche 1 bis 5 in Verbindung mit einem Antioxidationsmittel.

8. Pharmazeutische Formulierung aus einer wäßrigen Lösung von Dobutamin-(+)-tartrat und Natriumbisulfit.

9. Pharmazeutische Formulierung aus einer wäßrigen Lösung von Dobutaminlactobionat.

10. Pharmazeutische Formulierung aus einer wäßrigen Lösung von Dobutaminlactat.

11. Verfahren zur Herstellung eines Salzes von Dobutamin mit einer Säure der Formel I

$$(OH)_{1-8}(C_{2-11}\ Alkyl)(COOH)_{1-2} \qquad I$$

oder mit Lactobionsäure oder Glycolsäure, dadurch gekennzeichnet, daß

(a) Dobutamin als freie Base mit der Säure der Formel (I) neutralisiert wird oder

(b) ein Säureadditionssalz von Dobutamin einer metathetischen Reaktion mit einem Salz der Säure der Formel (I) unterzogen wird.

**Patentansprüche für den Vertragsstaat: AT**

1. Verfahren zur Herstellung eines Salzes von Dobutamin mit einer Säure der Formel I

$$(OH)_{1-8}(C_{2-11}\ Alkyl)(COOH)_{1-2} \qquad I$$

oder mit Lactobionsäure oder Glycolsäure, dadurch gekennzeichnet, daß

(a) Dobutamin als freie Base mit der Säure der Formel (I) neutralisiert wird oder

(b) ein Säureadditionssalz von Dobutamin einer metathetischen Reaktion mit einem Salz der Säure der Formel (I) unterzogen wird.

2. Verfahren nach Anspruch 1, worin die Säure der Formel (I) Weinsäure ist.

3. Verfahren nach Anspruch 2, worin die Säure der Formel (I) (+)-Weinsäure ist.

4. Verfahren nach Anspruch 1, worin die Säure Lactobionsäure ist.

5. Verfahren nach Anspruch 1, worin die Säure Milchsäure ist.

**Revendications pour les Etats contractants: BE CH LI DE FR GB IT LU NL SE**

1. Sel de la N-[3-(4-hydroxyphényl)-1-méthylpropyl]-2-(3,4-dihydroxyphényl)éthylamine avec un acide de formule I:

$$(OH)_{1-8}(alkyl\ en\ C_{2-11})(COOH)_{1-2}$$

ou bien avec l'acide lactobionique ou l'acide glycolique.

2. Tartrate de dobutamine.

3. (+)-tartrate de dobutamine.

4. Lactobionate de dobutamine.

5. Lactate de dobutamine.

6. Base libre de dobutamine filtrable, cristalline, solide.

7. Formulation pharmaceutique comprenant une solution aqueuse d'un sel de dobutamine selon l'une quelconque des revendications 1 à 5, en association avec un antioxydant.

8. Formulation pharmaceutique comprenant une solution aqueuse de (+)-tartrate de dobutamine et du bisulfite de sodium.

9. Formulation pharmaceutique comprenant une solution aqueuse de lactobionate de dobutamine.

10. Formulation pharmaceutique comprenant une solution aqueuse de lactate de dobutamine.

11. Procédé pour la préparation d'un sel de dobutamine et acide de formule:

I

ou bien l'acide lactobionique ou l'acide glycolique, ce procédé consistant à:

(a) neutraliser la dobutamine comme base libre avec l'acide, ou

(b) soumettre un sel d'addition d'acides de dobutamine à une réaction de double décomposition avec un sel de l'acide.

**Revendications pour l'Etat contractant: AT**

1. Procédé pour la préparation d'un sel de dobutamine et acide de formule:

$$(OH)_{1-8}(alkyl\ en\ C_{2-11})(COOH)_{1-2}$$

ou bien avec l'acide lactobionique ou l'acide glycolique, ce procédé consistant à:

(a) neutraliser la dobutamine comme base libre avec l'acide, ou

(b) soumettre un sel d'addition d'acides de dobutamine à une réaction de double décomposition avec un sel de l'acide.

2. Procédé selon la revendication 1, dans lequel l'acide de formule (I) est l'acide tartrique.

3. Procédé selon la revendication 2, dans lequel l'acide de formule (I) est l'acide (+)-tartrique.

4. Procédé selon la revendication 1, dans lequel l'acide est l'acide lactobionique.

5. Procédé selon la revendication 1, dans lequel l'acide est l'acide lactique.